# EUROPEAN PATENT APPLICATION

(11) **EP 1 782 809 A1**
(43) Date of publication of application: **09.05.2007**
(21) Application number: 05020264.7
(22) Date of filing: 16.09.2005
(51) Int. Cl.: A61K 31/4439, A61P 11/00

(54) **Use of ligands of PPARGamma for inducing foetal lung maturity and the prevention of the respiratory-distress syndrome in premature infants**

(71) Applicant: Charité-Universitätsmedizin Berlin, 10117 Berlin (DE)
(72) Inventor: Guthmann, Florian, 12207 Berlin (DE); Rüstow, Bernd, 13465 Berlin (DE)
(74) Representative: Krauss, Jan

(57) **Abstract**

The present invention relates to the use of ligands (such as agonists and antagonists) of PPARγ in the prevention and treatment of respiratory-distress syndrome in premature infants.

### Annex to the application documents - subsequently filed sequences listing

SEQUENCE LISTING

<110> Charité - Universitätsmedizin Berlin

<120> Use of ligands of PPARγ for inducing foetal lung maturity and the
prevention of the respiratory-distress syndrome in premature
infants

<130> FB15815

<140> EP05020264.7-2123
<141> 2005-09-16

<160> 6

<170> PatentIn version 3.4

<210> 1
<211> 25
<212> DNA
<213> Rattus norvegicus

<400> 1
tacagatggg acatggaatg atagg 25

<210> 2
<211> 25
<212> DNA
<213> Rattus norvegicus

<400> 2
agggttcatc ttttcgtcta attgc 25

<210> 3
<211> 22
<212> DNA
<213> Rattus norvegicus

<400> 3
cctacccttg aagctgcttg tg 22

<210> 4
<211> 25
<212> DNA
<213> Rattus norvegicus

<400> 4
gtttaatctg gccctggaag tagtc 25

<210> 5
<211> 24
<212> DNA
<213> Rattus norvegicus

<400> 5
tccactggca tcgttctata tgac 24

<210> 6
<211> 26
<212> DNA
<213> Rattus norvegicus

<400> 6
gagccatctt catgatgtag cagtag 26

## Description

The present invention relates to the use of ligands (such as agonists and antagonists) of PPAR_{γ} in the prevention and treatment of respiratory-distress syndrome in premature infants. For the purposes of the present invention, all references are incorporated by reference in their entireties.

### Description

The efforts for a better medical care of premature infants have led to a drastic decrease of neonatal mortality (1. to 28. day after birth). In Germany, the mortality of premature infants has decreased by approximately 85 % since 1970, and, until the 90ties, this had the largest impact on the decrease of the overall mortality of newborns in Germany.

This development took place for several reasons:
- the medical care of high-risk pregnancies in perinatal centres,
- consequent induction of the lung development using corticosteroids,
- birth of premature infants in perinatal centres,
- (early) surfactant-therapy,
- shorter duration and milder forms of respiration or support of breathing, and
- postnatal steroid therapy (declining since 1997; [1])

### The respiratory-distress syndrome is caused by a lack of surfactant

In 1959, Avery and Mead [27] showed that the pathophysiological basis of the respiratory-distress syndrome of premature and neonatal infants is a lack of lung surfactant.

Surfactant is a lipid-rich material that covers the inner surface of the alveoli as a monomolecular layer ("monolayer"). The surfactant-specific main component DPPC, together with specific proteins, is responsible for the reduction of the surface tension at the air-water-boundary and thereby avoids the collapse of the alveoli in the end-expiration phase. In the last trimester of foetal development the production of surfactant strongly increases. Nevertheless, the amount and quality of surfactant only shortly before birth (in humans approx. after 34 weeks of pregnancy) reach values that provide a sufficient adaptation of the lung the external life. From this, it can be explained that premature infants born before 34 weeks of gestation frequently develop a life-threatening respiratory-distress syndrome (also known as hyaline-membrane disease) that has its cause in a lack of surfactant caused by an immature lung.

Synthesis, storage and secretion of the surfactant are a specific output of the type II cells that make up about 15 % of the approximately 40 different cell types in the lung [2;3].

### Induction of the maturation of the lung with corticosteroids

In 1968, Liggins found that the lungs of premature born lambs surprisingly contained air after antenatal gavage of dexamethasone [4]. He concluded that dexamethasone could induce the synthesis of surfactant. Ever since, the morphological and biochemical development of the lung is intensively studied.

A milestone during the biochemical maturation of the lung is the occurrence of lamellar bodies after 22 to 24 weeks of pregnancy (human). Lamellar bodies constitute the storage form of the surfactant. They are synthesised in type II pneumocytes and released into the alveoli where the surfactant then spreads out as a monomolecular layer. The role of the individual components of the surfactant is not fully understood, yet. Nevertheless, genetic defects of ABCA3 and surfactant protein B which are lethal and of surfactant protein C which cause severe lung disease shall be mentioned here:
ABCA3; Shulenin and co-workers described a mutation of ABCA3 in newborn that died because of a therapy refractory respiratory-distress syndrome [5]. ABCA3 is a transporter protein in the membrane of lamellar bodies whose expression is inducible by corticosteroids - an evidence for its importance in the synthesis of surfactant that is also inducible by corticosteroids.

Surfactant protein B; More than 27 "loss of function" mutations of the SP-B-gene were described that lead to a lethal respiratory-distress syndrome in newborns (review in [6]). SP-B is required for the synthesis of normal lamellar bodies, the reduction of surface tension at the air-liquid interface and for the formation of lungs and tubular myelin, as well as the prote-proteolytic processing of SP-C. Thus, SP-B-deficient mice and humans are also deficient in SP-C. SP-B, therefore, plays a critical role in surfactant homeostasis required for lung function in the neonatal and adult lung [6a]. Regarding the development of a respiratory-distress syndrome, SP-B appears to be the most important apoprotein of the surfactant.

Surfactant protein C; SP-C increases surfactant spreading and stability and is a major component of surfactant preparations routinely used in neonatology. Deletion of SP-C (SP-C -/mice) disturbed surfactant stability and caused severe lung disease but did not cause respiratory failure at birth. Although lamellar body formation was seen in SP-C -/- mice, inflammation, remodeling, and abnormal lipid accumulations occured within the lungs of SP-C-deficient mice. Likewise, SP-C deficiency was associated with severe interstitial lung disease in a family lacking mature SP-C [6a].

### Clinical research

The importance of the induction of the maturation of the lung is undisputed. The antenatal (maternal) administration of steroids (induction of the maturation of the lung) reduces the incidence of the respiratory distress syndrome and the intraventricular bleeding. In particular, it reduces the mortality of premature infants having a birth weight below 1500 g (VLBW) [7;8]. In 1996, in six large neonatal intensive care unitas in Germany, 76 % of the premature infants (gestation age 27-32 weeks) received a prenatal induction of the maturation of the lung [9]. Whilst in the year 1991 only 24 % of the mothers received steroids before birth (Vermont Oxford Network database), in 1999 in the same clinics 75 % of the mothers received steroids [1]. The increased use of this effective prophylaxis is one of the most important reasons for the decreasing mortality of premature infants.

### Risks and side-effects of the antenatal administration of steroids

Epidemiological data from humans are scarce, but animal experiments render it more and more likely that the antenatal administration of corticosteroids leads to side-effects. On the one hand there appears to be a connection between foetal growth and later occurring diseases, such as coronary heart disease, type II diabetes, dyslipidaemia, and hypertonus (Barker-hypothesis). There are numerous evidences from animal experiments for a causal connection between an administration of antenatal corticosteroid and reduced glucose tolerance and hypertonia in adult animals [10-12]. On the other hand, the antenatal administration of corticosteroid also affects neurological development:. Matthews et al. have shown that both endoge-endogenous glucocorticoid and synthetic glucocorticoid exposure has a number of rapid effects in the foetal brain in late gestation, including modification of neurotransmitter systems and transcriptional machinery. Such foetal exposure permanently alters hypothalamo-pituitary-adrenal (HPA) function in prepubertal, postpubertal, and aging offspring, in a sex-dependent manner [14a].

Antenatal treatment with corticosteroid drugs has been found to adversely impact many physiological systems in the developing monkey, especially if multiple injection paradigms were utilized and when the Dexamethasone treatment was sustained for 2 or more weeks. In contrast, single injections particularly at low doses did not seem to cause undesired effects of the same magnitude, although the efficacy of such a brief and limited treatment for enhancing airway functioning is questionable. The one systematic dose/response study conducted in the rhesus monkey led the investigators to recommend using a dose of 0.5 mg/kg of maternal body weight, administered 4 times at 12 h intervals to stimulate lung maturation [14b]. They concluded that this regime would be efficacious for improving respiratory function, while likely limiting the side effects of corticosteroid treatments.

However, even with doses scaled to match those used in clinical treatment of humans [14c] side effect occurred. Examination of the brains of the foetuses at end of treatment indicated that there was a marked reduction in the frontal cortical region of cytoskeletal microtubule associated proteins (MAPs) as well as in a presynaptic marker protein, synaptophysis, similar to what had been observed in the foetal sheep following beta-methasone exposure ([for review see [14d]). In addition, two administrations of steroids does reduce the growth of the brain in premature and term sheep [13; 14]

The advantages and disadvantages of the steroid prophylaxis of the respiratory-distress syndrome must carefully be considered. The use of the steroid prophylaxis by a similarly potent substance having lower risks would be desirous for the clinic. Possibly, also the combination of steroid and another substance could be considered.

The induction of the maturation of the lung should reduce the incidence and/or the severity of the respiratory-distress syndrome and lower the mortality of premature infants.

It is therefore an object of the present invention to provide an improved prevention and therapy of the respiratory-distress syndrome, in particular in premature infants.

This object is solved according to the present invention by a use of a ligand of PPARγ for the production of a medicament for the prevention or treatment of a disease that is related to a lack of surfactant in the lung.

The present invention thus relates to the use of ligands (such as agonists and antagonists) of PPARγ as a medicament for the treatment of the respiratory-distress syndrome and for the induction of the foetal maturation of the lung in order to prevent and/or treat the respiratory-distress syndrome in premature infants and newborns.

Surprisingly, the present inventors were able to show that ligands of the gamma-subtype of the peroxisome-proliferator-activated-receptor (PPARγ), and in particular pioglitazone, were able to induce the biochemical maturation of the lung in the foetus in vivo upon antenatal administration.

Peroxisome proliferator-activated receptors (PPARs) are members of the nuclear hormone receptor superfamily of ligand-activated transcription factors that are related to retinoid, steroid and thyroid hormone receptors. The PPAR subfamily comprises three members: PPAR-α, PPAR-β, and PPAR-γ. Two isoforms of PPAR-γ are known: PPAR-γ1 and PPAR-γ2. PPAR-γ1 is the major isoform and accounts for approximately 85% of PPARs in adipose tissue. These isoforms are generated from the same gene by mRNA splicing and differ in their amino-terminal end, with PP AR-γ2 having an additional 30 amino acids. The name PPAR is derived from the fact that activation by xenobiotics of PPAR-β results in peroxisome proliferation in rodent hepatocytes. Activation of PPAR-β or PPAR-γ, however, does not elicit this response. Most tissues in humans (and rodents) express all three receptor subtypes, although there is considerable variability in the relative expression. PPARs regulate gene expression by binding, as heterodimers with retinoid X receptors (RXRs), to specific PPAR response elements (PPRE) in the promoter regions of specific target genes, resulting in either the activation or suppression of a specific gene.

The recent development of a novel class of insulin-sensitising drugs, the thiazolidinediones (TZDs, i.e. rosiglitazone, pioglitazone), which are synthetic PPAR-gamma agonists, repre-represents a significant advance in antidiabetic therapy. Another member of the TZDs, troglitazone, has been withdrawn from the market due to its hepatotoxicity.

In the context of the present invention, the terms "ligand" or "ligands" of PPAR_{γ} relate to compounds and/or molecules that bind to PPAR_{γ} and influence the biological function of PPAR_{γ}. Ligands can be agonists or antagonists of the biological function of PPAR_{γ}. The ligands can either bind directly to the active site of the PPAR_{γ}-receptor or to a position that sterically influences said active site. Furthermore, the ligand can bind in combination with a co-factor, such as a second chemical entity, a peptide, protein, or the like. Many ligands of the PPAR_{γ}-receptor are already known and, without limitation, include the TZDs, for example, rosiglitazone, ciglitazone, and pioglitazone, as well as the cyclopentanone prostaglandins 15D-PGJ2 (15-deoxy-delta12,14-prostaglandin J2) and PGA1. Other possible ligands could be lipopolysaccharides (LPS).

In earlier published work of the inventors ("Phenotype of palmitic acid transport and of signalling in alveolar type II cells from E/H-FABP double-knockout mice: contribution of caveolin-1 and PPARy" in Biochimica et Biophysica Acta 1636, 2004,196-204) the effective compound pioglitazone was also used as ligand of PPAR_{γ} in knock-out mice. Nevertheless, the present invention is completely different, since the present induction of the maturation of the lung is essentially different from the examination of the lipid turnover in type II - alveolar cells. In the examples as described in the above publication, the lipid turnover could only be increased up to the level of the control organisms by direct administration of pioglitazone. In contrast, in the present invention, an antenatal treatment with pioglitazone led to an unexpected acceleration of the expression of proteins which are related to lung maturation in the foetus.

Preferably, the disease to be prevented or treated is caused by an immature lung in a premature infant. More preferably, said disease is respiratory-distress syndrome, in particular in premature infants and/or newborns.

Preferably, the ligand of PPARγ to be used according to the present invention is selected from the group of thiazolidinediones, such as, for example, rosiglitazone, ciglitazone, and pioglitazone, cyclopentanone prostaglandins, such as 15D-PGJ2 or PGA1, and lipopolysaccharides (LPS).

More preferably, the medicament containing the ligand of PPAR_{γ} is applied antenatally.

Ligands of PPARγ, such as thiazolidindiones or glitazones represent a novel class of active compounds for the treatment of type-2-diabetes. They are described as insulin sensitizer or insulin reactivators, since they increase the sensitivity against insulin in glucose consuming tissues. The ligand of PPARγ, pioglitazone, (Actos®) induces the biochemical maturation of the foetal lung.

The use according to the present invention provides the following advantages:
+ potential alternative to treatments with corticosteroids having side-effects,
+ active agent is already available, and
+ first data regarding side-effect free effectiveness in vivo (rats) exist.

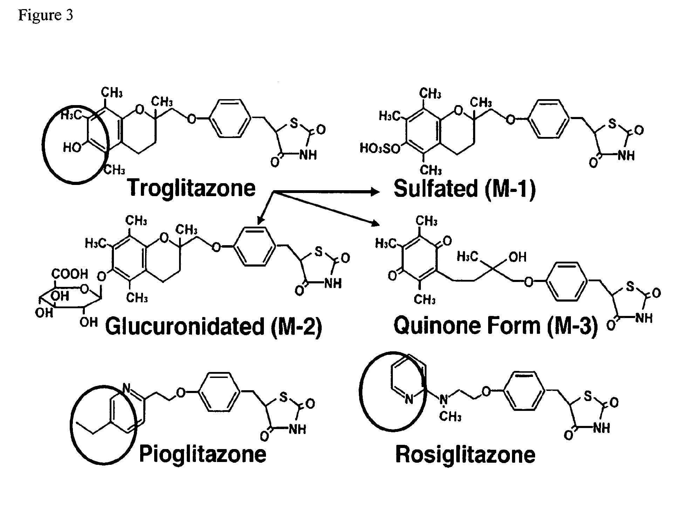

However, it has been noted that individual PPAR agonists exhibit toxic side-effects which are shortly reviewed.
Troglitazone differs from pioglitzone and rosiglitazone in a hydroxyl-group (Figure 3, circle). Troglitazone can be sulfatised at this site leading to a metabolite which can not be derrived from pioglitazone or rosiglitazone. Thus, it can be explained, why in controlled clinical studies the incidence of elevated transaminases was similar in the pioglitazone and rosiglitazone group, compared to the placebo group. In contrast, in patients that received troglitazone, the incidence of elevated transaminases was triplicated [19]. A less good parameter in order to compare the toxicity of the three glitazones is the number of casuistics. But also in this case, numerous reports regarding troglitazone are standing against rosiglitazone or two regarding pioglitazone. In addition, the causality for the latter substances could not be shown in a patient with a high incidence of liver disease, adipositas, insulin resistance, and diabetes type 2. Two casuistics for pioglitazone are present, wherein in one case the liver values before the treatment were not measured [20] (see also the commentary [21]) and in the other case the symptomatics after stopping of pioglitazone was fully reversible [22].

It could be shown in clinical studies that ligands of PPARγ in women with polycystic ovaries lead to ovulatory cycles, and can significantly improve the untreated extremely high infertility of these patients improve [23]. Due to the often existing lack of clarity regarding the date of such pregnancies and the possible exposition of the foetuses with ligands of PPAR_{γ}, there is increasing interest to obtain data regarding ligands of PPAR_{γ} in the pregnancy. The inventors are aware of two casuistics that describe the uptake of glitazones during unknown pregnancies. In both cases, healthy children were born [24,25].

In rats, rosiglitazone had no effect on the implantation or embryogenesis. Even the 20 or 75-fold of the maximal recommended human dosage had no teratogenic effect in rats or rabbits. In the late pregnancy, this dosis can slow down the foetal growth, possibly this points to an accelerated differentiation of cells [26].

In another embodiment of the present invention the medicament that is used according to the present invention is administered by various routes, for example oral, parenteral, subcutaneous, intramuscular, intravenous or intracerebral. The preferred route of administration would be parenterally at daily doses of the compound for adult human treatment of about 0.01 -5000 mg, preferably 1-1500 mg per day. Preferably, said medicament is applied in a dosage of between 30 mg/d and 2000 mg/d, preferably between 100 mg/d and 1600 mg/d, most preferred between 300 to 800 mg/d. The appropriate dose may be administered in a single dose or as divided doses presented at appropriate intervals for example as two, three, four or more subdoses per day.

Appropriate dosages can be readily obtained by a person of skill through routine experimentation, and can be are based on factors such as the concentration of the active ingredient, the body weight and age of the patient, and other patient or active ingredient related factors. In the context of the present invention, the following considerations might be employed in order to extrapolate an appropriate dosage from the animal tests that were performed. In general, the transfer of data from rat to human is difficult and should be done with caution. However, with regard to the morphological development of lung, there are two similarities between rat and human. Rat gestational day 19.5 is in the second half of the canalicular period which corresponds to a human gestational age of 20 to 24 weeks. On the other hand, lamellar bodies (LBs) first occur at day 19 which corresponds to human gestational age of 24 weeks. Thus, rat gestational day 19.5 matches (20 to) 24 weeks of human gestation. This is a stage of gestation where resucitation of the premature infant is thought to be reasonable and where survival rates increase clearly. Thus, the inventors conclude that the time point chosen in their animal model conclude that the time point chosen in their animal model reflects a phase of human gestation which is of clinical interest.

| | Rat days | % | Human days | % |
|---|---|---|---|---|
| Gestational length | 22 | 100 | 280 | 100 |
| Glandular period until | 18 | 80 | 112 | 40 |
| Canalicular period | 19-20 | 86-91 | 112-168 | 40-60 |
| Saccular period from | 21 | 95 | 168 | 60 |
| Appearance of LBs¹ | 19 | 86 | 165 | 60 |

| | | | | |
|---|---|---|---|---|
| ¹ The stage where mature lamellar bodies start to increase rapidly is listed. Data from Bourbon, JR, Fraslon, C. Developmental aspects of the alveolar epithelium and the pulmonary surfactant system. in: Pulmonary Surfactant: Biochemical, Functional, Regulatory, and Clinical Concepts. Ed. Jacques R. Bourbon. 1991; CRC press Boca Raton. | | | | |

For preparing pharmaceutical compositions containing ligands of the invention, inert, pharmaceutically acceptable carriers are used. The pharmaceutical carrier can be either solid or liquid. Solid form preparations include, for example, powders, tablets, dispersible granules, capsules, cachets, and suppositories. A solid carrier can be one or more substances which can also act as diluents, flavouring agents, solubilizers, lubricants, suspending agents, binders, or tablet disintegrating agents; it can also be an encapsulating material. In powders, the carrier is generally a finely divided solid which is in a mixture with the finely divided active component. In tablets, the active compound is mixed with the carrier having the necessary binding properties in suitable proportions and compacted in the shape and size desired.

Powders and tablets preferably contain between about 5% to about 70% by weight of the ligand as the active ingredient. Suitable carriers include, for example, magnesium carbonate, magnesium stearate, talc, lactose, sugar, pectin, dextrin, starch, tragacanth, methyl cellulose, sodium carboxymethyl cellulose, a low-melting wax, cocoa butter and the like.

The pharmaceutical compositions can include the formulation of the ligand with an encapsulating material as a carrier providing a capsule in which the ligand (active component; with or without other carriers) is surrounded by a carrier, which is thus in association with it. In a similar manner, cachets are also included. Tablets, powders, cachets, and capsules can be used as solid dosage forms suitable for oral administration.

Liquid pharmaceutical compositions include, for example, solutions suitable for oral or parenteral administration, or suspensions, and emulsions suitable for oral administration. Sterile water solutions of the active component or sterile solutions of the active component in solvents comprising water, ethanol, or propylene glycol are examples of liquid compositions suitable for parenteral administration.

Sterile solutions can be prepared by dissolving the active component in the desired solvent system, and then passing the resulting solution through a membrane filter to sterilize it or, alternatively, by dissolving the sterile compound in a previously sterilized solvent under sterile conditions.
Pharmaceutical compositions generally are administered in an amount effective for treatment or prophylaxis of a specific condition or conditions. Initial dosing in human is accompanied by clinical monitoring of symptoms, such symptoms for the selected condition. In general, the compositions are administered in an amount of active agent of at least about 100 µg/kg body weight. In most cases they will be administered in one or more doses in an amount not in excess of about 20 mg/kg body weight per day. Preferably, in most cases, doses is from about 100 µg/kg to about 5 mg/kg body weight, daily.

For administration particularly to mammals, and particularly humans, it is expected that the daily dosage level of active agent will be 0,1 mg/kg to 10 mg/kg and typically around 1 mg/kg. By "therapeutically effective amount" is meant a symptom-alleviating or symptom - reducing amount of a ligand to be used according to the invention. Thus, PPAR_{γ} ligand (optionally in combination with RXR selective agonists) contemplated for use in the practice of the present invention can be employed for both in vitro and in vivo applications. For in vivo applications, the above-described ligands can be incorporated into a pharmaceutically acceptable formulation for administration, as above. Those of skill in the art can readily determine suitable dosage levels when compounds contemplated for use in the practice of the present invention are so used.

In accordance with another embodiment of the present invention, there are provided compositions comprising at least one PPARγ ligand (as described herein) and at least one retinoid X receptor (RXR) selective agonist, optionally in a pharmaceutically acceptable carrier. Exemplary pharmaceutically acceptable carriers include carriers suitable for oral, intravenous, subcutaneous, intramuscular, intracutaneous, and the like administration, as described above. Administration in the form of creams, lotions, tablets, dispersible powders, granules, syrups, elixirs, sterile aqueous or non-aqueous solutions, suspensions or emulsions, and the like, is contemplated.

For the preparation of oral liquids, suitable carriers include emulsions, solutions, suspensions, syrups, and the like, optionally containing additives such as wetting agents, emulsifying and suspending agents, sweetening, flavouring and perfuming agents, and the like. For the preparation of fluids for parenteral administration, suitable carriers include sterile aqueous or non-aqueous solutions, suspensions, or emulsions. Examples of non-aqueous solvents or vehicles are propylene glycol, polyethylene glycol, vegetable oils, such as olive oil and corn oil, gelatine, and injectable organic esters such as ethyl oleate. Such dosage forms may also contain adjuvants such as preserving, wetting, emulsifying, and dispersing agents. They may be sterilized, for example, by filtration through a bacteria-retaining filter, by incorporating sterilizing agents into the compositions, by irradiating the compositions, or by heating the compositions. They can also be manufactured in the form of sterile water, or some other sterile injectable medium immediately before use.

Another aspect of the present invention relates to a method for screening for drug candidates (ligands) for modulating PPARγ-receptor activity. US 6,821,737 describes method and kits for screening for transcription factor modulators efficiently. In one aspect, the method can be employed to profile multiple, different transcription factors activated in a sample in the presence of each of the transcription factor modulators. Comparison of the activated transcription factor profiles in the presence and absence of the modulator identifies the transcription factor modulator that modulates the activation of specific transcription factors. The person of skill can suitably modify the method as described in US 6,821,737 in order to screen for ligands of the PPAR_{γ}-receptor.

Another aspect of the present invention relates to a use according to the present invention, wherein said medicament is applied in combination with corticosteroids and/or RXR selective compound (agonist). Preferably, said retinoid X receptor (RXR) selective compound is selected from a substituted benzoic acid or derivative thereof, a substituted nicotinic acid or derivative thereof or a substituted carboxylated furan or LG268.

Another aspect of the present invention relates to a method for treating a subject suffering from a disease state which is the result of an immature lung, said method comprising administering to said subject an amount of a therapeutic composition effective to ameliorate the effect of said immature lung, wherein said therapeutic composition comprises at least one PPAR_{γ}-ligand in a pharmaceutically acceptable carrier therefore. Other embodiments relating to the method according to the present invention are as described above.

It should be understood that the features of the invention as disclosed and described herein can be used not only in the respective combination as indicated but also in a singular fashion without departing from the intended scope of the present invention.

The invention will now be described in more detail by reference to the following Figures, and the Examples. The following examples are provided for illustrative purposes only and are not intended to limit the invention.

**Figure 1** shows the expression of different apoproteins of the surfactant in foetuses (d 19,5) that received antenatal pioglitazone (black bars) or placebo (white bars). Median ± SD of n=2 independent experiments.

**Figure 2** shows the expression of the transport protein ABCA3 (red) that is localised in the lamellar bodies. Histology of the foetal lung (d 19,5) following antenatal feeding with placebo (left side) or feeding containing the substance to be examined (pioglitazone; right side). The nuclei are DAPI-labelled (blue). Sections of 3 lungs of n=2 independent experiments.

**Figure 3** shows the formula of troglitazone and its most important metabolites.

### Examples

### Methods

The inventors examined the effect of the antenatally applied ligands of PPARγ pioglitazone on the expression of the surfactant-apoproteins A, B and C and on the expression of the transporter ABCA3 that is critical for the surfactant-synthesis.

### Feeding Regime

Wistar rats, bred in our animal facilities, received standard diet or a diet supplemented with 0.25 g/kg of pioglitazone (ssniff Spezialdiäten GmbH, Soest, Germany) ad libitum from day 14.5 to 19.5 of gestation, with day 0 corresponding to the day when the vaginal smear contained sperm (term = 22 days). The rats were killed at day 18.5 or 19.5 of gestation by diethyl ether excess, and the fetuses were removed from the mothers.

### Fluorescence microscopy

The foetal lungs were dissected out and fixed with 3% neutral buffered formalin for 48 hours. Fixed lungs were paraffin-embedded and cut into sections of 4 µm. Then, they were deparaffinized in xylene and rehydrated in graded alcohol solutions. The sections were permeabilized in 0.01 M PBS (pH 7.5; containing 1% (w/v) BSA and 0.3% (w/v) Triton X-100) for 1h at room temperature and immuno-stained with specific antibodies direct against ABCA3, an integral lamellar body-limiting membrane protein (clone 3C9, Covance/Berkeley Antibody, Richmond, CA, USA), followed by Alexa 488-labeled anti-mouse-IgG (Molecular Probes). Cell nuclei were labelled with DAPI. CLSM was performed using a ZEISS LSM 510 META with Axiovert 200 M (Carl Zeiss Jena GmbH, Jena, Germany). Images were taken using a Plan-Apochromat 63x/1.4 oil immersion objective and fluorescence excitation at 543 nm (1 mW HeNe-Laser) and 488 nm (30 mW Ar-Laser).

### Reverse transcriptase and real-time quantitative PCR

Total RNA was isolated using the RNeasy Mini Kit (Qiagen, Hilden, Germany) following the manufacturer's instructions. Samples were further treated with DNase I (DNA-free; Ambion, Austin, TX, USA) to avoid any DNA contamination. Quality of RNA was assessed by formaldehyde agarose gel electrophoresis. Reverse transcription was performed with 0.5 µg RNA added to the reaction mix containing AmpliTaq Gold DNA polymerase, RNase inhibitor, AmpliTaq DNA polymerase, and random hexamers (Applied Biosystems, Foster City, CA, USA). Negative reverse transcriptase (RT) (no enzyme) and no-template (no RNA) controls were also included. The RT thermal cycle was 10 min at 25 °C, 60 min at 37 °C, and 5 min at 95 °C. For real-time PCR, SP-A, SP-B and SP-C were detected using SYBR Green. Primers are shown in Table 1. Primers were purchased from Metabion (Martinsried, Germany). Primers were designed with Primerdesigner (Applied Biosystems). The correct product size was then confirmed by electrophoresis. The PCR mixture (25 µl total volume) consisted of 0.3 µM each primer, 2x reaction buffer (dNTP, HotGoldstar DNA polymerase, uracil-N-glycosylase, 5 nM MgCl2), SYBR Green, and 1 µl of template. Amplification and detection were performed using Taqman (Applied Biosystems) with the following cycle profile: 2 min at 50 °C, 10 min at 95 °C, and 40 cycles of 15 s at 95 °C + 1 min at 60 °C. The primers used were as follows:
SP-A
   TACAGATGGGACATGGAATGATAGG (5'FORWARD)
   AGGGTTCATCTTTTCGTCTAATTGC (5'REVERSE);
SP-B
   CCTACCCTTGAAGCTGCTTGTG (5'FORWARD)
   GTTTAATCTGGCCCTGGAAGTAGTC (5'REVERSE);
SP-C
   TCCACTGGCATCGTTCTATATGAC (5'FORWARD)
   GAGCCATCTTCATGATGTAGCAGTAG (5'REVERSE)

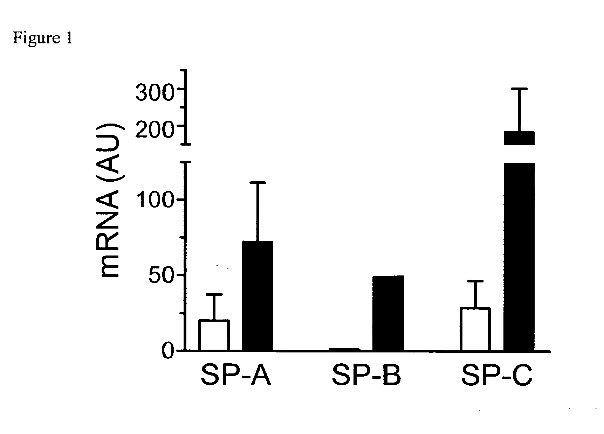

Pioglitazone (antenatal) leads to a drastic increase of the expression of the SP-A, SP-B and SP-C-encoding mRNA (Figure 1). The increase is detectable at day 19.5 but was not observed at day 18.5 of gestation (not shown). Particularly pronounced is the effect in SP-B the possibly most important apoprotein of the surfactant for the development of the respiratory distress-syndrome.

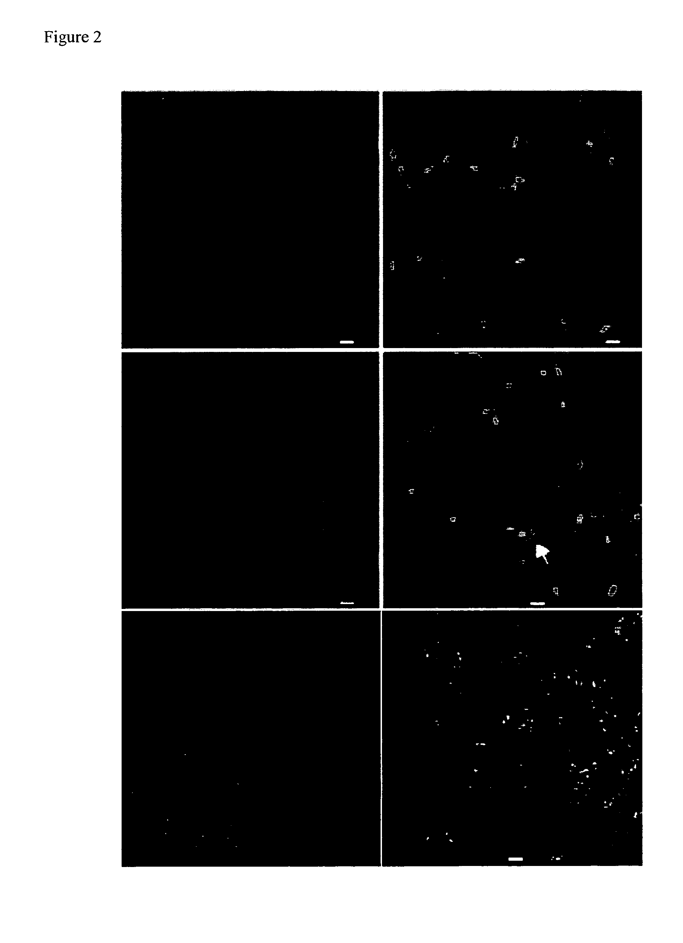

ABCA3 is expressed in the rat in a development-specific manner, and is detected earliest at day 20.5, i.e. shortly before birth by immunoblotting [15]. By the more sensitive RT-PCR, the inventors could detect a trace of ABCA3 expression at day 19.5. Following antenatal administration of pioglitazone, the expression of ABCA3 (red) can clearly be detected (Figure 2).

The inventors follow from the increased expression of three apoproteins of the surfactant and ABCA3, a marker protein of lamellar bodies and a transport protein being important for the synthesis of surfactant that the antenatal administration of pioglitazone can induce the biochemical maturation of the lung. The inventors expect from the induction of the maturation of the lung - in analogy to the corticosteroids - a reduction of the mortality of premature infants associated with respiratory distress-syndrome.

### Cited literature

1. Horbar JD, Badger GJ, Carpenter JH, Fanaroff AA, Kilpatrick S, LaCorte M et al. Trends in mortality and morbidity for very low birth weight infants, 1991-1999. Pediatrics 2002; 110(1 Pt 1):143-151.
2. Crapo JD, Peters-Golden M, Marsh-Salin J, Shelburne JS. Pathologic changes in the lungs of oxygen-adapted rats: a morphometric analysis. Lab Invest 1978; 39(6):640-653.
3. Haies DM, Gil J, Weibel ER. Morphometric study of rat lung cells. 1. Numerical and dimensional characteristics of parenchymal cell population. Am Rev Respir Dis 1981; 123(5):533-541.
4. Liggins GC. Premature delivery of foetal lambs infused with glucocorticoids. J Endocrinol 1969; 45(4):515-523.
5. Shulenin S, Nogee LM, Annilo T, Wert SE, Whitsett JA, Dean M. ABCA3 gene mutations in newborns with fatal surfactant deficiency. N EngI J Med 2004; 350(13):1296-1303.
6. Gortner L, Hilgendorff A. [Surfactant-associated proteins B and C: molecular biology and physiologic properties]. Z Geburtshilfe Neonatol 2004; 208(3):91-97.
6a. Whittsett JA, Wert, SE, Xu, Y. Genetic disorders of surfactant homeostasis. Biol Neonate 2005; 87:283-287.
7. Anonymous. Effect of corticosteroids for foetal maturation on perinatal outcomes. NIH Consensus Development Panel on the Effect of Corticosteroids for Foetal Maturation on Perinatal Outcomes. JAMA 1995; 273(S):413-418.
8. Crowley PA. Antenatal corticosteroid therapy: a meta-analysis of the randomized trials, 1972 to 1994. Am J Obstet Gynecol 1995; 173(1):322-335.
9. Gortner L, Wauer RR, Stock GJ, Reiter HL, Reiss l, Jorch G et al. Neonatal outcome in small for gestational age infants: do they really better? J Perinat Med JID - 0361031 1999; 27(6):484- 489.
10. Clark PM. Programming of the hypothalamo-pituitary-adrenal axis and the foetal origins of adult disease hypothesis. EurJ Pediatr 1998; 157 SuppI 1:S7-10.:S7-10.
11. Dodic M, Wintour EM, Whitworth JA, Coghian JP. Effect of steroid hormones on blood pressure. Clin Exp Pharmacol Physiol 1999; 26(7):550-552.
12. Edwards LJ, Coulter CL, Symonds ME, McMillen IC. Prenatal undernutrition, glucocorticoids and the programming of adult hypertension. Clin Exp Pharmacol Physiol 2001; 28(11):938-941.
13. Jobe AH, Wada N, Berry LM, Ikegami M, Ervin MG. Single and repetitive maternal glucocorticoid exposures reduce foetal growth in sheep. Am J Obstet Gynecol 1998; 178(5):880-885.
14. Huang WL, Beaziey LD, Quinlivan JA, Evans SF, Newnham JP, Dunlop SA. Effect of corticosteroids on brain growth in foetal sheep. Obstet Gynecol 1999; 94(2):213-218.
14a. Matthews, SG, Owen, D, Kalabis, G, Banjanin, S, Setiawan, EB, Dunn, EA, Andrews, MH. Foetal glucocorticoid exposure and hypothalamo-pituitary-adrenal (HPA) function after birth. Endocr-Res. 2004 Nov; 30(4): 827-36
14b. Engle, MJ, Kemnitz, JW, Yao, TJ, Perelman, RH, Farell, PM. Effects of maternal dexamethasone therapy on foetal lung development in the rhesus monkey. Am J Perinatol 1996; 13:399-407.
14c. Antonow-Schlorke, I, Schwab, M, Li, C, Nathanielsz, PW. Glucocorticoid exposure at the dose used clinically alters cytoskeletal proteins and presynaptic terminals in the foetal baboon brain, J Physiol 2003; 547:117-123.
14d. Coe, CL, Lubach, GR. Developmental consequences of antenatal dexamethasone treatment in nonhuman primates. Neurosci Biobehav Rev 2005; 29:227-235
15. Yoshida I, Ban N, Inagaki N. Expression of ABCA3, a causative gene for fatal surfactant deficiency, is up-regulated by glucocorticoids in lung alveolar type II cells. Biochem Biophys Res Commun 2004; 323(2):547-555.
16. Serenius F, Ewald U, Farooqi A, Holmgren PA, Hakansson S, Sedin G. Short-term outcome after active perinatal management at 23-25 weeks of gestation. A study from two Swedish tertiary care centres. Part 1: maternal and obstetric factors. Acta Paediatr 2004; 93(7):945-953.
17. He K, Talaat RE, Pool WF, Reily MD, Reed JE, Bridges AJ et al. Metabolie activation of troglitazone: identification of a reactive metabolite and mechanisms involved. Drug Metab Dispos 2004; 32(6):639-646.
18. Nozawa T, Minami H, Sugiura S, Tsuji A, Tamai l. Role of organic anion transporter OATP1B1 (OATP-C) in hepatic uptake of irinotecan and its active metabolite, 7-ethyl-10-hydroxycamptothecin: in vitro evidence and effect of single nucleotide polymorphisms. Drug Metab Dispos 2005; 33(3):434-439.
19. Scheen AJ. Hepatotoxicity with thiazolidinediones: is it a class effect? Drug Saf 2001; 24(12):873-888.
20. Farley-Hills E, Sivasankar R, Martin M. Fatal liver failure associated with pioglitazone. BMJ 2004; 329(7463):429.
21. Amin P. Liver toxicity and pioglitazone: data are missing. BMJ 2004; 329(7471):918.
22. May LD, LefkowitchJH, Kram MT, Rubin DE. Mixed hepatocellular-cholestatic liver injury after pioglitazone therapy. Ann Intern Med 2002; 136(6):449-452.
23. Azziz R, Ehrmann D, Legro RS, Whitcomb RW, Hanley R, Fereshetian AG et al. Trogiitazone improves ovulation and hirsutism in the polycystic ovary syndrome: a multicenter, double blind, placebo-controlled trial. J Clin Endocrinol Metab 2001; 86(4):1626-1632.
24. Kalyoncu N1, Yaris F, UIku C, Kadioglu M, Kesim M, Unsal M et al. A case of rosiglitazone exposure in the second trimester of pregnancy. Reprod Toxicol 2005; 19(4):563-564.
25. Yaris F, Yaris E, Kadioglu M, UIku C, Kesim M, Kalyoncu NII. Normal pregnancy outcome following inadvertent exposure to rosiglitazone, glidazide, and atorvastatin in a diabetic and hypertensive woman. Reprod Toxicol 2004; 18(4):619-621.
26. Chan LY, Yeung JH, Lau TK. Placental transfer of rosiglitazone in the first trimester of human pregnancy. Fertil Steril 2005; 83(4):955-958.
27. Avery ME, Mead J. Surface properties in relation to atelectasis and hyaline membrane disease. AMA J Dis Child. 1959 May;97(5, Part 1):517-23.

## Claims

1. Use of a ligand of PPAR_{γ} for the production of a medicament for the prevention or treatment of a disease that is related to a lack of surfactant in the lung.

2. Use according to claim 1, wherein said disease is caused by an immature lung in a premature infant.

3. Use according to claim 1 or 2, wherein said disease is respiratory-distress syndrome.

4. Use according to any one of claims 1 to 3, wherein said ligand is selected from the group of thiazolidinediones, such as, for example, rosiglitazone, ciglitazone, and pioglitazone, cyclopentanone prostaglandins, such as 15D-PGJ2 or PGA1, and lipopolysaccharides (LPS).

5. Use according to any one of claims 1 to 4, wherein said medicament is applied antenatally.

6. Use according to any one of claims 1 to 5, wherein said medicament is applied orally or parenterally.

7. Use according to any one of claims 1 to 6, wherein said medicament is applied in a dosage of between 30 mg/d and 2000 mg/d, preferably between 100 mg/d and 1600 mg/d, most preferred between 300 to 800 mg/d.

8. Use according to any one of claims 1 to 7, wherein said medicament is applied in combination with corticosteroids and/or RXR selective agonists.

9. Use according to claim 8, wherein said retinoid X receptor (RXR) selective agonist is selected from a substituted benzoic acid or derivative thereof, a substituted nicotinic acid or derivative thereof or a substituted carboxylated furan or LG268.
